# EUROPÄISCHE PATENTSCHRIFT

(11) **EP 2 866 592 B1**
(45) Veröffentlichungstag und Bekanntmachung des Hinweises auf die Patenterteilung: **27.07.2016**
(21) Anmeldenummer: 12733056.1
(22) Anmeldetag: 28.06.2012
(51) Int. Cl.: A23L 35/00, A23P 10/00, A61K 47/10, A61K 9/00, A61K 47/32, A61K 47/36

(54) **VERFAHREN ZUR HERSTELLUNG EINER FOLIENFORMULIERUNG FÜR EINE ESSBARE FOLIE UND DEREN VERWENDUNG**
METHOD FOR PRODUCING A FILM FORMULATION FOR AN EDIBLE FILM, AND THE USE THEREOF
PROCÉDÉ DE FABRICATION D'UNE COMPOSITION POUR UNE FEUILLE COMESTIBLE ET UTILISATION DE LADITE FEUILLE

(43) Veröffentlichungstag der Anmeldung: 06.05.2015
(73) Patentinhaber: Optimags Dr. Zimmermann Gmbh, 76149 Karlsruhe (DE)
(72) Erfinder: ZIMMERMANN, Laura, 76131 Karlsruhe (DE)
(74) Vertreter: Lemcke, Brommer & Partner Patentanwälte Partnerschaft mbB
(86) Internationale Anmeldenummer: PCT/EP2012/062605
(87) Internationale Veröffentlichungsnummer: WO 2014/000803

(56) Entgegenhaltungen:
- EP-A2- 1 676 557
- WO-A1-2004/035029
- WO-A2-2009/045022
- US-A1- 2010 240 724

## Beschreibung

Die vorliegende Erfindung betrifft ein Verfahren zur Herstellung einer Folienformulierung für eine essbare Folie und eine essbare Folie sowie deren Verwendung.

Essbare Folien werden beispielsweise verwendet, um Lebensmittelzusatzstoffe in exakter Dosierung für Lebensmittel verarbeitende Betriebe bereitzustellen, beispielsweise Lebensmittelfarbstoffe, Gewürze, Enzyme sowie Zutaten für Kaltmixgetränke. Diese Lebensmittelzusatzstoffe werden direkt auf die essbare Folie dosiert und mit dieser zusammen den Lebensmitteln zugegeben. Die essbare Folie löst sich dabei innerhalb von normalerweise einer Minute in Wasser vollständig auf.

Neben dem eben genannten Einsatzzweck werden essbare Folien in jüngster Zeit auch dazu verwendet, bioaktive Substanzen, insbesondere Medikamente in exakter Dosierung oral zu verabreichen. Dies ist auch das bevorzugte Anwendungsgebiet der vorliegenden Erfindung. Hierzu wird der medizinische Wirkstoff auf eine essbare Folie aufgebracht und gegebenenfalls mit einer zweiten Folie abgedeckt. Der Wirkstoff wird aufgenommen, indem die mit der bioaktiven Substanz versehene essbare Folie in den Mund genommen wird und dort verbleibt, bis sich die essbare Folie aufgelöst hat.

Medikamente, die oral zu verabreichen sind, werden bislang in der Regel als Tabletten oder Kapseln verabreicht. Um die erwünschte medizinische Wirkung zu entfalten, müssen die Wirkstoffe allerdings in sehr hoher Genauigkeit dosiert werden, wobei viele Wirkstoffe nur in geringsten Mengen von wenigen Mikrogramm zugeführt werden dürfen.

Andererseits müssen Tabletten und Kapseln jedoch eine gewisse Mindestgröße bzw. Mindestmasse aufweisen, um für die zu behandelnden Personen handhabbar zu sein. Dementsprechend werden die medizinischen Wirkstoffe zur Herstellung von Tabletten oder Kapseln mit Füllstoffen und Bindemitteln in beträchtlicher Menge versetzt. Diese Stoffe müssen sowohl für den Organismus als auch für den medizinischen Wirkstoff verträglich sein, so dass deren Herstellung und Bereitstellung bereits einen beträchtlichen Kostenfaktor bei der Herstellung des fertigen Medikaments darstellt. Des Weiteren ist es notwendig, den medizinischen Wirkstoff und die Zuschlagsstoffe bzw. Bindemittel vollständig zu durchmischen, was aufwendige Maschinen notwendig macht. Schließlich ist es für eine erfolgreiche Therapie oft wünschenswert, kleinere Wirkstoffeinheiten oral zu verabreichen, als diejenige Wirkstoffmenge, die in einer Tablette oder Kapsel enthalten ist. Das Teilen von Tabletten ist jedoch kaum in der gewünschten und gegebenenfalls sogar erforderlichen Genauigkeit möglich.

Vor diesem Hintergrund gibt es seit einiger Zeit auch essbare Folien, die als Trägermaterial für den medizinischen Wirkstoff dienen können. Die Dosierung des medizinischen Wirkstoffs ist dabei in geringsten Mengen und in hoher Genauigkeit möglich, da er feinst verteilt auf die Oberfläche der essbaren Folie aufgebracht werden kann. Die Flächeneinheiten der essbaren Folie entsprechen dann sehr exakt einer spezifischen Dosierung des medizinischen Wirkstoffs. Beispiele für solche essbaren Folien sind beispielsweise in der US 2004/0136923A1 und in der EP 1 306 071 B1 zu finden. Auch in der DE 10 2010 009 071 A1 ist eine essbare Folie und ein Verfahren zu deren Herstellung offenbart.

Für unterschiedliche Anwendungszwecke, insbesondere zur oralen Verabreichung von Medikamenten, ist es wünschenswert, in die essbaren Folien Inhaltsstoffe auf öliger Basis einzubringen, beispielsweise ätherische Öle, die den Geschmack der essbaren Folie positiv beeinflussen.

Soweit essbare Folie mit Inhaltsstoffen auf öliger Basis länger gelagert werden, ergibt sich bei essbaren Folien nach dem Stand der Technik allerdings das Problem, dass diese Inhaltsstoffe nach einiger Zeit der Lagerung wieder aus der essbaren Folie austreten oder agglomerieren. Hierdurch fühlt sich die Folienoberfläche dann nicht mehr trocken an, was von den Anwendern als minderwertig empfunden werden könnte. Des Weiteren könnten Ölagglomerate die exakte Gleichverteilung des Gewichts der essbaren Folie über deren Fläche beeinträchtigen, was insbesondere bei medizinischen Anwendungen Probleme bereiten kann.

Essbare Folien, die nach einem Verfahren der vorliegenden Art hergestellt werden, werden in der Regel maschinell verarbeitet, geprüft, in Stücke geschnitten und verpackt. Hierfür muss das Folienmaterial eine entsprechende mechanische Belastbarkeit aufweisen. In diesem Zusammenhang muss darauf geachtet werden, dass die essbare Folie keine Lufteinschlüsse enthält. Bislang musste das Herstellen einer Folienformulierung für eine essbare Folie daher zumindest teilweise unter Vakuum erfolgen, was naturgemäß den Aufwand für die Herstellung der Folienformulierung erhöhte.

Aufgabe der vorliegenden Erfindung ist es daher, ein Herstellungsverfahren für essbare Folien bereitzustellen, in die ölige Komponenten stabil eingelagert werden können und die trotz unaufwendiger Herstellung eine hohe mechanische Stabilität aufweisen.

Die Aufgabe der vorliegenden Erfindung wird durch ein Verfahren zur Herstellung einer Folienformulierung für eine essbare Folie gelöst, das die folgenden Schritte umfasst:
a) Herstellung einer Lösung A durch Rühren bei einer Temperatur im Bereich zwischen 20 ° bis 100 °C, wobei die Lösung A Stärke, Polyethylenglykol, Glycerin, Polyvinylalkohol, mindestens einen Entschäumer, Zucker, mindestens eine ölige Komponente, mindestens einen Emulgator und Wasser als Lösungsmittel enthält,
b) Homogenisieren und Temperieren der in Schritt (a) erhaltenen Lösung auf eine Temperatur von mindestens 70 °C, vorzugsweise auf 80 °C bis 85 °C,
c) Herunterkühlen der in Schritt (b) erhaltenen Lösung.

Als ölige Komponente können ätherische Öle, wie beispielsweise Pfefferminzöl, eingesetzt werden, die beispielsweise als Geschmackstoffe in der essbaren Folie dienen. Mit der vorliegenden Erfindung ist es jedoch auch möglich, ölige pharmazeutische Wirkstoffe in einer essbaren Folie einzubinden.

Durch die Verwendung eines Emulgators, der die ölige Komponente dispergiert, wird langfristig verhindert, dass es zu einem Austritt der öligen Komponente aus der essbaren Folie oder zu einer Agglomerierung kommt.

Zudem verhindert der Entschäumer eine Bläschenbildung, so dass das erfindungsgemäße Verfahren in vielen Fällen mit guten Ergebnissen unter normalem Umgebungsdruck durchgeführt werden kann.

In einer bevorzugten Ausführungsform der Erfindung umfasst die Herstellung der Lösung A die folgenden Schritte:
α) Herstellung einer Lösung B durch Rühren bei einer Temperatur im Bereich zwischen 20 ° bis 100 °C, wobei die Lösung B Polyethylenglykol, Glycerin, Polyvinylalkohol, mindestens einen Entschäumer, Zucker, mindestens eine ölige Komponente, mindestens einen Emulgator und Wasser als Lösungsmittel enthält,
β) Herstellung einer Lösung C durch Lösen von Stärke in Wasser bei Raumtemperatur,
γ) Einrühren der Lösung B in Lösung C.

In einer weiter bevorzugten Ausführungsform der Erfindung umfasst die Herstellung der Lösung A oder die Herstellung der Lösung B die folgenden Schritte:
i) Auflösen von Polyethylenglykol und Glycerin in siedendem Wasser,
ii) Homogenisieren der in Schritt (i) erhaltenen Lösung,
iii) Hinzufügen von Polyvinylalkohol-Granulat unter Rühren,
iv) Homogenisieren der in Schritt (iii) gewonnen Lösung,
v) Hinzufügen eines Entschäumers zu der Lösung,
vi) Hinzufügen von Zucker zu der Lösung,
vii) Vermengen mindestens einer öligen Komponente mit mindestens einem Emulgator in einem separaten Behälter und
viii) Hinzufügen des in Schritt (vii) erhaltenen Substanzgemisches zu der in den Schritten (i) bis (vi) erhaltenen Lösung.

Durch ein solches Verfahren wird die Herstellung von essbaren Folien ermöglicht, die eine auch nach langer Lagerung trockene und gleichmäßige Filmoberfläche aufweisen.

Das Homogenisieren erfolgt vorzugsweise in einem Rührkessel bei 10000 bis 20000 Umdrehungen/min, insbesondere bei 16000 Umdrehungen/min, wodurch die Qualität, insbesondere die Gleichmäßigkeit der Filmoberfläche, der essbaren Folien, die aus der Folienformulierung hergestellt werden, verbessert wird.

Vorzugsweise wird der Schritt (vii) bei Raumtemperatur durchgeführt, um die Qualität der eingesetzten öligen Komponente nicht zu beeinträchtigen.

In einer bevorzugten Ausführungsform der Erfindung wird die Folienformulierung für eine essbare Folie aus
- 9 - 16 Gew./%: Stärke
- 5 - 10 Gew./%: Polyethylenglykol
- 0,1 - 0,5 Gew./%: Emulsionsstabilisator
- 10 - 15 Gew./%: Polyvinylalkohol
- 4 - 6 Gew./%: Glycerin
- 3 - 6 Gew./%: Zucker
- 0,001 - 0,2 Gew./%: Entschäumer
- 0,1 - 0,15 Gew./%: Emulgator
- 7 - 10 Gew./%: ölige Komponente
- 35 - 50 Gew./%: Wasser
hergestellt. Als Stärke hat sich die Verwendung von Reisstärke als vorteilhaft erwiesen. Ein geeigneter Emulsionsstabilisator (bzw. Verdichtungsmittel) ist Hydroxypropylmethylcellulose (HPMC E3). Als Entschäumer und als Emulgator werden modifizierte Polydimethoxysilane eingesetzt.

Des Weiteren wird die Aufgabe der vorliegenden Erfindung durch eine essbare Folie gelöst, wobei die essbare Folie aus einer Folienformulierung, die gemäß dem erfindungsgemäßen Herstellungsverfahren hergestellt wurde, hergestellt wird, also mindestens eine ölige Komponente enthält. Eine solche essbare Folie weist eine gleichmäßige Filmoberfläche auf und ist trocken, da die öligen Komponenten durch die Verwendung eines Emulgators und eines Entschäumers an einem Austreten oder Agglomerieren in der essbaren Folie gehindert werden. Zudem enthält eine solche essbare Folie keine oder nur sehr geringe Lufteinschlüsse, wodurch die Biegsamkeit der Folie erhöht und deren Brüchigkeit verringert wird. Nicht zuletzt wird hierdurch auch ein noch genaueres Dosieren von Inhaltsstoffen der essbaren Folie ermöglicht.

Die erfindungsgemäße essbare Folie wird zweckmäßigerweise in einer Dicke von 60 µm bis 100 µm gefertigt.

Weiterhin wird die Aufgabe der vorliegenden Erfindung durch eine Verwendung der erfindungsgemäßen essbaren Folie zur oralen Applikation von bioaktiven Substanzen gelöst. Die essbare Folie wird dabei als Trägermedium für die orale Verabreichung von bioaktiven Substanzen und besonders bevorzugt von Medikamenten verwendet.

Des Weiteren wird die Aufgabe der vorliegenden Erfindung durch eine Verwendung der erfindungsgemäßen essbaren Folie zur Dosierung von Lebensmittelzusatzstoffen gelöst.

Es ist aber auch denkbar weitere Wirkstoffe oder Hilfsstoffe, wie beispielsweise Geschmacks- oder Farbstoffe, in die essbare Folie einzubringen.

Im Folgenden wird die Erfindung anhand eines Beispiels näher beschrieben.

Als Rohstoffe zur Herstellung einer Folienformulierung für eine essbare Folie werden beispielsweise die folgenden Rohstoffe verwendet:

| Rohstoffe | prozentualer Anteil (in Gew.%) |
|---|---|
| Reisstärke | 9 - 16 |
| Polyethylenglykol 1000 | 5 - 10 |
| HPMC e3 | 0,1 - 0,5 |
| Polyvinylalkohol (Mowiol^{®} 4-88) | 10 - 15 |
| Glycerin | 4 - 6 |
| Zucker | 3 - 6 |
| Entschäumer: mod. Polydimethylsiloxan | 0,001 - 0,2 |
| Emulgator: mod. Polydimethylsiloxan | 0,1 - 0,15 |
| ölige Komponente: z.B. Pfefferminzöl | 7 - 10 |
| Wasser | 35 - 50 |

Der Wassergehalt der erfindungsgemäßen essbaren Folie hängt von den Trocknungsbedingungen ab und kann variieren.

Ein Herstellungsverfahren einer Folienformulierung für eine essbare Folie, in der eine oder mehrere ölige Komponenten gebunden sind und beim Herstellungsprozess entstehende Luftblasen reduziert werden, kann zum Beispiel wie folgt ablaufen:
a) 50 % der Gesamtmenge an Wasser wird erhitzt, bis die Siedetemperatur erreicht ist.
b) In das siedende Wasser wird die erforderliche Menge Polyethylenglykol 1000 (PEG-1000) und Glycerin hinzugegeben und aufgelöst. Danach wird die Lösung in einen Rührkessel mit Homogenisator bei 16000 U/min eingefüllt.
c) Unter ständiger Zirkulation wird in die noch heiße Lösung die erforderliche Menge Polyvinylalkohol (Mowiol^{®} 4-88) hinzugefügt.
d) Diesem Gemisch wird als Entschäumer, ein modifiziertes Polydimethylsiloxan, hinzugefügt.
e) Anschließend wird die benötigte Zuckermenge untergemischt.
f) Parallel wird in einem separaten Behältnis die erforderliche Menge der öligen Komponente (oder einem Gemisch aus mehrerer öliger Komponenten) mit dem Emulgator vermengt und der Lösung aus Schritt (e) hinzugefügt.
f) In einem weiteren Schritt wird parallel in einem separaten Behälter die Reisstärke mit den restlichen 50 % der Gesamtmenge des Wassers bei Raumtemperatur aufgelöst.
h) Die in Schritt (e) gewonnene Lösung wird in die Lösung aus Schritt (d) eingerührt. Unter Beibehaltung der Drehzahl aus Schritt (b) für den Homogenisator wird die Lösung auf eine Temperatur von 80 °C erhitzt. Bei Erreichen der gewünschten Temperatur ist die Stärke vollständig gebunden.
i) Abschließend wird das Gemisch auf 50 °C heruntergekühlt.
j) Abfüllen der essbaren Filmlösung, die eine oder mehrere ölige Komponenten und eine stark reduzierte Menge an Luftblasen enthält.

Abschließend wird die Folienformulierung abgekühlt und zu einer essbaren Folie verarbeitet.

Abhängig von der gewünschten Verwendung der Folienformulierung, kann diese zusätzlich zu den oben genannten Substanzen Arzneistoffe und/oder Hilfsstoffe, wie Geschmacksstoffe und/oder (Lebensmittel-)Farbstoffe enthalten.

## Patentansprüche

1. Verfahren zur Herstellung einer Folienformulierung für eine essbare Folie, folgende Schritte umfassend:
a) Herstellung einer Lösung A durch Rühren bei einer Temperatur im Bereich zwischen 20 ° bis 100 °C, wobei die Lösung A Stärke, Polyethylenglykol, Glycerin, Polyvinylalkohol, mindestens einen Entschäumer, Zucker, mindestens eine ölige Komponente, mindestens einen Emulgator und Wasser als Lösungsmittel enthält,
b) Homogenisieren und Temperieren der in Schritt (a) erhaltenen Lösung auf eine Temperatur von mindestens 70 °C, vorzugsweise auf 80 °C bis 85 °C,
c) Herunterkühlen der in Schritt (b) erhaltenen Lösung.

2. Verfahren nach Anspruch 1,
**dadurch gekennzeichnet,**
**dass** die Herstellung der Lösung A die folgenden Schritte umfasst:
α) Herstellung einer Lösung B durch Rühren bei einer Temperatur im Bereich zwischen 20 ° bis 100 °C, wobei die Lösung B Polyethylenglykol, Glycerin, Polyvinylalkohol, mindestens einen Entschäumer, Zucker, mindestens eine ölige Komponente, mindestens einen Emulgator und Wasser als Lösungsmittel enthält,
β) Herstellung einer Lösung C durch Lösen von Stärke in Wasser bei Raumtemperatur,
γ) Einrühren der Lösung B in Lösung C.

3. Verfahren nach einem der Ansprüche 1 oder 2,
**dadurch gekennzeichnet,**
**dass** die Herstellung der Lösung A oder die Herstellung der Lösung B die folgenden Schritte umfasst:
i) Auflösen von Polyethylenglykol und Glycerin in siedendem Wasser,
ii) Homogenisieren der in Schritt (i) erhaltenen Lösung,
iii) Hinzufügen von Polyvinylalkohol unter Rühren,
iv) Homogenisieren der in Schritt (iii) gewonnen Lösung,
v) Hinzufügen eines Entschäumers zu der Lösung,
vi) Hinzufügen von Zucker zu der Lösung,
vii) Vermengen mindestens einer öligen Komponente mit mindestens einem Emulgator in einem separaten Behälter und
viii) Hinzufügen des in Schritt (vii) erhaltenen Substanzgemisches zu der in den Schritten (i) bis (vi) erhaltenen Lösung.

4. Verfahren nach Anspruch 3,
**dadurch gekennzeichnet,**
**dass** das Homogenisieren in einem Rührkessel bei 10000 bis 20000 Umdrehungen/min erfolgt.

5. Verfahren nach Anspruch 3,
**dadurch gekennzeichnet,**
**dass** der Schritt (vii) bei Raumtemperatur durchgeführt wird.

6. Verfahren nach einem der vorangestellten Ansprüche,
**dadurch gekennzeichnet,**
**dass** die essbare Folienformulierung aus
9 - 16 Gew./% Stärke
5 - 10 Gew./% Polyethylenglykol
0,1 - 0,5 Gew./% Emulsionsstabilisator
10 - 15 Gew./% Polyvinylalkohol
4 - 6 Gew./% Glycerin
3 - 6 Gew./% Zucker
0,001 - 0,2 Gew./% Entschäumer
0,1 - 0,15 Gew./% Emulgator
7 - 10 Gew./% ölige Komponente
35 - 50 Gew./% Wasser
hergestellt wird.

7. Essbare Folie, hergestellt aus einer Folienformulierung, die mit einem Verfahren nach mindestens einem der vorangestellten Ansprüche erhalten wurde.

8. Essbare Folie nach Anspruch 7 zur oralen Applikation von bioaktiven Substanzen.

9. Essbare Folie nach Anspruch 8, **dadurch gekennzeichnet,**
**dass** die bioaktive Substanz ein Medikament ist.

10. Verwendung der essbaren Folien nach Anspruch 7 zur Dosierung von Lebensmittelzusatzstoffen.

## Claims

1. Method of preparing a film formulation for an edible film, comprising the following steps:
a) preparation of a solution A by stirring at a temperature in the range between 20° to 100°C, wherein solution A contains starch, polyethylene glycol, glycerol, polyvinyl alcohol, at least one antifoam, sugar, at least one oily component, at least one emulsifier and water as solvent,
b) homogenisation and tempering of the solution obtained in step (a) at a temperature of at least 70°C, preferably at from 80°C to 85°C,
c) cooling of the solution obtained in step (b).

2. Method according to claim 1,
**characterised in that**
the preparation of solution A comprises the following steps:
α) preparation of a solution B by stirring at a temperature in the range between 20° to 100°C, wherein solution B contains polyethylene glycol, glycerol, polyvinyl alcohol, at least one antifoam, sugar, at least one oily component, at least one emulsifier and water as solvent,
β) preparation of a solution C by dissolving starch in water at room temperature,
γ) introduction of solution B into solution C.

3. Method according to either one of claims 1 and 2,
**characterised in that**
the preparation of solution A or the preparation of solution B comprises the following steps:
i) dissolution of polyethylene glycol and glycerol in boiling water,
ii) homogenisation of the solution obtained in step (i),
iii) addition of polyvinyl alcohol with stirring,
iv) homogenisation of the solution obtained in step (iii),
v) addition of an antifoam to the solution,
vi) addition of sugar to the solution,
vii) mixing of at least one oily component with at least one emulsifier in a separate container and
viii) addition of the substance mixture obtained in step (vii) to the solution obtained in steps (i) to (vi).

4. Method according to claim 3,
**characterised in that**
the homogenisation is effected in a stirrer vessel at from 10,000 to 20,000 rev/min.

5. Method according to claim 3,
**characterised in that**
step (vii) is carried out at room temperature.

6. Method according to any one of the preceding claims,
**characterised in that**
the edible film formulation is prepared from
9 - 16 % by weight starch
5 - 10 % by weight polyethylene glycol
0.1 to 0.5 % by weight emulsion stabiliser
10 - 15 % by weight polyvinyl alcohol
4 - 6 % by weight glycerol
3 - 6 % by weight sugar
0.001 to 0.2 % by weight antifoam
0.1 - 0.15 % by weight emulsifier
7 - 10 % by weight oily component
35 - 50 % by weight water.

7. Edible film, prepared from a film formulation that has been obtained using a method according to at least one of the preceding claims.

8. Edible film according to claim 7 for oral administration of bioactive substances.

9. Edible film according to claim 8,
**characterised in that**
the bioactive substance is a medicament.

10. Use of the edible films according to claim 7 for dispensing doses of food additives.

## Revendications

1. Procédé de préparation d'une formulation de film pour un film comestible, comprenant les étapes suivantes :
a) préparation d'une solution A par brassage à une température dans la plage comprise entre 20 ° et 100 °C, sachant que la solution A contient de l'amidon, du polyéthylène glycol, de la glycérine, de l'alcool polyvinylique, au moins un agent antimoussant, du sucre, au moins une composante huileuse, au moins un émulsifiant et de l'eau comme agent de dissolution ou solvant,
b) homogénéisation et portée de la solution obtenue à l'étape (a) à une température d'au moins 70 °C, de préférence de 80 °C à 85 °C,
c) refroidissement de la solution obtenue à l'étape (b).

2. Procédé selon la revendication 1, **caractérisé en ce que** la préparation de la solution A comprend les étapes suivantes :
α) préparation d'une solution B par brassage à une température dans la plage comprise entre 20 ° et 100 °C, sachant que la solution B contient du polyéthylène glycol, de la glycérine, de l'alcool polyvinylique, au moins un agent antimoussant, du sucre, au moins une composante huileuse, au moins un émulsifiant et de l'eau comme agent de dissolution ou solvant,
β) préparation d'une solution C par dissolution d'amidon dans de l'eau à température ambiante,
γ) incorporation de la solution B dans la solution C par brassage.

3. Procédé selon la revendication 1 ou 2, **caractérisé en ce que** la préparation de la solution A ou la préparation de la solution B comprend les étapes suivantes :
i) dissolution de polyéthylène glycol et de glycérine dans de l'eau bouillante,
ii) homogénéisation de la solution obtenue à l'étape (i),
iii) adjonction d'alcool polyvinylique avec brassage,
iv) homogénéisation de la solution obtenue à l'étape (iii),
v) adjonction d'un agent antimoussant à la solution,
vi) adjonction de sucre à la solution,
vii) mélangeage d'au moins une composante huileuse avec au moins un émulsifiant dans un récipient séparé et
viii) adjonction du mélange de substances obtenu à l'étape (vii) à la solution obtenue aux étapes (i) à (vi).

4. Procédé selon la revendication 3, **caractérisé en ce que** l'homogénéisation s'effectue dans une cuve de brassage à 10 000 à 20 000 tours/minute.

5. Procédé selon la revendication 3, **caractérisé en ce que** l'étape (vii) est effectuée à température ambiante.

6. Procédé selon l'une des revendications précédentes, **caractérisé en ce que** la formulation de film comestible est préparée à partir de
9 à 16% en poids d'amidon
5 à 10% en poids de polyéther glycol
0,1 à 5% en poids de stabilisateur d'émulsion
10 à 15% en poids d'alcool polyvinylique
4 à 6% en poids de glycérine
3 à 6% en poids de sucre
0,001 à 0,2% en poids d'agent antimoussant
0,1 à 0,15% en poids d'émulsifiant
7 à 10% en poids de composante huileuse
35 à 50% en poids d'eau.

7. Film comestible, fabriqué à partir d'une formulation de film qui a été obtenue avec un procédé selon au moins une des revendications précédentes.

8. Film comestible selon la revendication 7 pour l'application orale de substances bioactives.

9. Film comestible selon la revendication 8, **caractérisé en ce que** la substance bioactive est un médicament.

10. Utilisation des films comestibles selon la revendication 7 pour le dosage d'additifs alimentaires.
